# EUROPEAN PATENT APPLICATION

(11) **EP 4 311 826 A1**
(43) Date of publication of application: **31.01.2024**
(21) Application number: 22187548.7
(22) Date of filing: 28.07.2022
(51) Int. Cl.: C07C 43/178, C11D 1/00, C07C 43/13, C07H 1/00, A61Q 19/00

(54) **BIOBASED SURFACTANT**

(71) Applicant: ECOLE POLYTECHNIQUE FEDERALE DE LAUSANNE (EPFL), 1015 Lausanne (CH)
(72) Inventor: Bertella, Stefania, 1018 Lausanne (CH); Komarova, Anastasiia, 1022 Chavannes près Renens (CH); Sun, Songlan, 1024 Ecublens (CH); Luterbacher, Jeremy, 1022 Chavannes près Renens (CH)
(74) Representative: Schaad, Balass, Menzl & Partner AG

(57) **Abstract**

The present invention relates to a compound of the general formula (I), and (II) 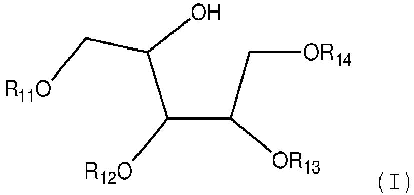 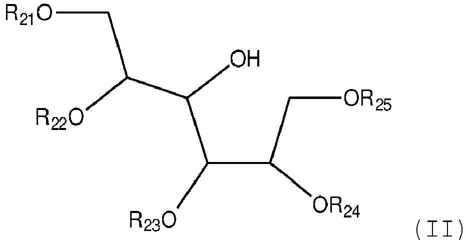 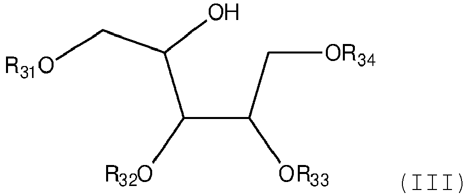 wherein one of R₁₁ and R₁₂ is hydrogen and the other is -CH₂-R₇₀, and one of R₁₃ and R₁₄ is hydrogen and the other is -CH₂-R₇₁, or wherein one of R₁₁ and R₁₂ is hydrogen and the other is -CH₂-R₇₀, and both of R₁₃ and R₁₄ are hydrogen, and wherein two of R₂₁, R₂₂ and R₂₃ is hydrogen and the other is -CH₂-R₇₀, and one of R₂₄ and R₂₅ is hydrogen and the other is -CH₂-R₇₁, wherein R₃₁, R₃₂ and R₃₃ are hydrogen and R₃₄ is -CH₂-R₇₁, and R₇₀ and R₇₁ are independently form each other and are selected from the group consisting of a linear or branched C₅ to C₂₀ alkyl, a (C₂ to C₁₀)-alkyloxy-(C₂ to C₁₀)-alkyl, a C₅ to C₁₀ cycloalkyl and C₆ to C₁₂ aryl.

## Description

The present invention relates to new compounds and their use as biobased surfactants.

Surfactants are a class of chemicals used in a wide range of applications and fields such as the detergent, medical, pharmaceutical, food and paint industries. With the advent of the COVID-19 global pandemic, the demand of surfactants has risen, due to the fact that these compounds contained in soaps and sanitizing compositions have the ability of disrupting the lipidic membrane of the SARS-CoV-2 virus making said virus inefficient.

Given the huge market and big demand for surfactants, it is important that their production does not rely only on fossil-based sources, but more and more it is fundamental that they are synthesized and sourced from renewable feedstocks that don't impact as much on the second current global challenge, climate change.

Moreover, as the lifetime of surfactant is short, given that they are generally washed away and tend to end up in the open, their structure had degradability products have to be compatible with the environment they end up in, so that they neither accumulate nor cause harm to the diverse species and ecosystems.

Bio-based surfactants (Sophorolipids, Rhamnolipids) are commercially available. Sophorolipids are commercialised by Ecover^{™}, Saraya^{™}, Intobio^{™}, Evonik^{™} and Allied Carbon Solutions^{™}. All of them have a critical micelle concentration CMC 7-10-fold less than CMC of SDS, but need to be produced with yeasts (average fermentations times: 7 days) and with fatty acids of tropical plant origin, that still pose an environmental pressure, due to deforestation issues.

US2021353517A1 discloses a process for producing a bio-based surfactant comprising an alkyl disulphate salt comprises the steps of methanolysis of medium chain length polyhydroxyalkanoic acid (mcl-PHA) to provide hydroxy fatty acid methyl ester monomers (HFAME's), reduction of the HFAME's to provide 1,3 alkyl diols, sulphation of the 1,3 alkyl diols to provide 1,3 alkyl disulphates, and neutralisation of the alkyl disulphates to provide a bio-based surfactant comprising 1,3 alkyl disulphate salt.

Particularly, carbohydrates (or their derivatives)-based surfactants have received a lot of attention and development for the variety of possible chemical reactions that are possible on the hydroxyl group of their core structure. Typical carbohydrate-based molecules or derivatives that can be chemically reacted to form surfactants are xylose, glucose, sorbitol, sorbitan, arabinose, isosorbide and uronic acid. Amongst the possible reaction that have been studied in making sugar-based surfactants the most common are the esterification of carbohydrate hydroxyl group with long chain acids, such as the case of the commercial Span and Tween derived from sorbitol. Other important reactions of carbohydrates to produce surfactants are etherification to form simple ether or reactions with long-chain aldehydes to form stable acetals. Finally, in the case of uronic acid, amide-based surfactants can be synthesized. Some of these reactions, although successful (such as the case of commercial esters based on sorbitan), are based on sugar or derivatives that undergo several synthetic steps of reduction, isomerization or dehydration of more abundant sugars before becoming surfactants, increasing their environmental and energetical impact with each synthetic step.

The problem of the present invention is therefore to provide bio-based surfactant which can be synthesized in a few steps directly from renewable sources.

The problem is solved by the compounds according to claim 1. Further preferred embodiments are subject of the dependent claims.

As explained in detail below, compounds of formula I and II can be obtained based on reductive hydrogenolysis of acetal stabilized carbohydrate-based molecules from biomass fractionation. The compounds of the present invention are biodegradable and have no negative impact on human and animal health. In addition, they have no or only a very limited negative influence on fauna, flora and ecosystems since they are derived from renewable resources. Furthermore, the synthesis of the compounds according to the present invention is simple which allows a large-scale bio-based surfactant production.

The present invention relates to a compound of the general formula (I), (II) and (III) 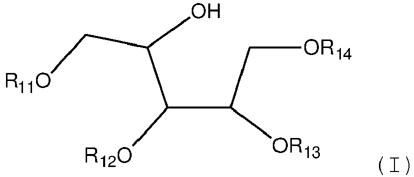 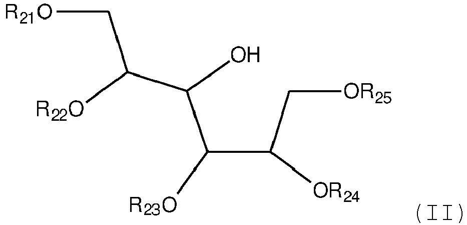 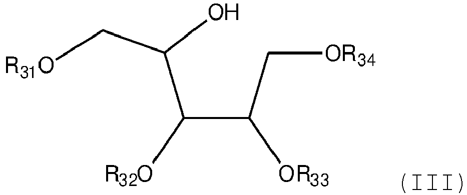
wherein one of R₁₁ and R₁₂ is hydrogen and the other is -CH₂-R₇₀, and one of R₁₃ and R₁₄ is hydrogen and the other is -CH₂-R₇₁, or wherein one of R₁₁ and R₁₂ is hydrogen and the other is -CH₂-R₇₀, and both of R₁₃ and R₁₄ are hydrogen, and
wherein two of R₂₁, R₂₂ and R₂₃ is hydrogen and the other is -CH₂-R₇₀, and one of R₂₄ and R₂₅ is hydrogen and the other is -CH₂-R₇₁, and
wherein R₃₁, R₃₂ and R₃₃ are hydrogen and R₃₄ is -CH₂-R₇₁, and
R₇₀ and R₇₁ are independently form each other and are selected from the group consisting of a linear or branched C₅ to C₂₀ alkyl, a (C₂ to C₁₀) -alkyloxy- (C₂ to C₁₀) -alkyl, C₅ to C₁₀ alkenyl, a C₃ to C₁₀ cycloalkyl, cycloalkylalkyl, cycloalkylalkenyl, and C₆ to C₁₂ aryl.

The term "linear or branched C₅ to C₂₀ alkyl" as a residue refers to a linear or branched hydrocarbon chain containing 5 to 20 of carbon atoms.

The term "a (C₁ to C₁₀) -alkyloxy- (C₁ to C₁₀)-alkyl" as a residue refers to a residue -(C₁-C₁₀)-O-(C₁-C₁₀)-, i.e., a linear or branched hydrocarbon chain containing preferably 1 to 20 of carbon atoms, of which at least two are singularly bonded to oxygen. Examples of said term as used herein include 2-ethylethoxy, 2-ethylpropoxy, 2-ethylpropoxy, 1-ethylbutoxy, 3-ethylbutoxy, and 2-ethylpropoxy.

The term "linear or branched C₅ to C₂₀ alkenyl" as a residue refers to a linear or branched hydrocarbon chain containing 2 to 20 of carbon atoms which contains at least one double bond.

The term "C₆ to C₁₂ aryl" refers to an aromatic carbon ring system having any suitable number of ring atoms and any suitable number of rings. Aryl groups can include any suitable number of carbon ring atoms, such as, 6, 7, 8, 9, 10, 11 or 12 ring atoms, as well as from 6 to 10, 6 to 12, or 6 to 14 ring members. Aryl groups can be monocyclic, fused to form bicyclic or tricyclic groups, or linked by a bond to form a biaryl group. Representative aryl groups include phenyl, naphthyl and biphenyl. Some aryl groups have from 6 to 12 ring members, such as phenyl, naphthyl or biphenyl. Other aryl groups have from 6 to 10 ring members, such as phenyl or naphthyl. Some other aryl groups have 6 ring members, such as phenyl.

The term "cycloalkyl" means a non-aromatic monocyclic ring system comprising 3 to 10 carbon atoms, preferably 5 to 10 carbon atoms. Preferred cycloalkyl rings contain about 5 to about 7 ring atoms. Non-limiting examples of suitable monocyclic cycloalkyls include cyclopentyl, cyclohexyl, cycloheptyl and the like.

The term "cycloalkylalkyl" means a residue -RₐR_{b} where Ra is an alkylene group having 1 to 8 carbon atoms and Rb is cycloalkyl group as defined above.

The term "cycloalkylalkenyl" means a residue -Rₐ'R_{b} where Rₐ' is an alkenylene group having 2 to 8 carbon atoms and R_{b} is cycloalkyl group as defined above.

The claimed compounds are stable at high pH and sensitive to low pH where they can hydrolyze and subsequently degrade to furans and humins. Therefore, they can be used as surfactants in cleaning under mild conditions, such as in personal care, dish-washing liquids, etc., but also for the applications where alkaline conditions are required.

The physico-chemical behavior of this compounds in oil-water systems are different from that of conventional non-ionics surfactants, since they have a pronounced lipophobic character compared to, for example, polyoxyethylene-based surfactant.

Another advantage of these compounds is their favorable environmental profile due to ability of the sugar core to be easily biodegraded while having a low toxicity.

Additionally, these compounds exhibit favorable dermatological properties, such as being very mild to skin and eye. This makes these compounds attractive for the use as surfactants in personal care products and technical applications such as emulsifiers, dispersants, surface modifiers or for solubilization especially in a pH range between 3 and 11.

Another advantage of these compounds is that they are suitable to work in hard water conditions with up to 180mg CaCO₃/L.

Additionally, these compounds are also suitable to work in very high pH condtions up to pH 14 for at least one hour.

In a preferred embodiment of the present invention compound of Compound of the general formula (I) and (II) according to Claim 1, wherein R₇₀ and R₇₁ are the same. Said compounds are synthetically easily available.

One embodiment of the present invention relates compound of the general formula (Ia) 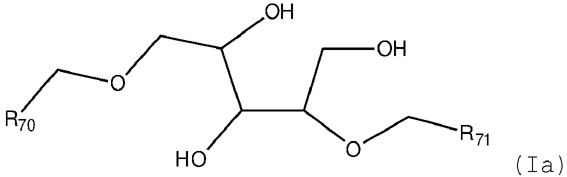 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂)-alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (Ib) 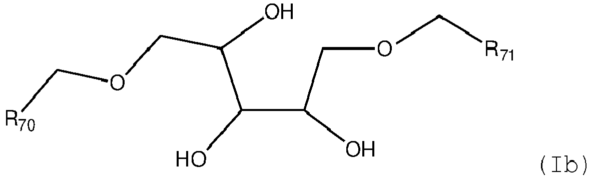 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (Ic) 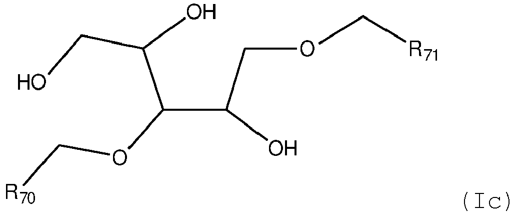 wherein R₇₀ an R₇₁ are selected from the group consisting of a linear or branched C₅ to C₂₀ alkyl, a (C₂ to C₁₀) -alkyloxy- (C₂ to C₁₀)-alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂)-alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (Ic) 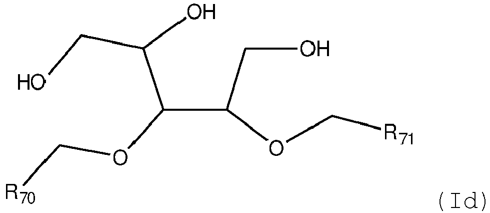 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIa) 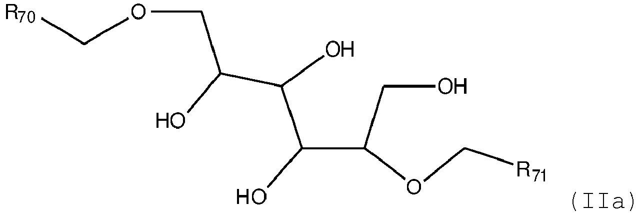 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIb) 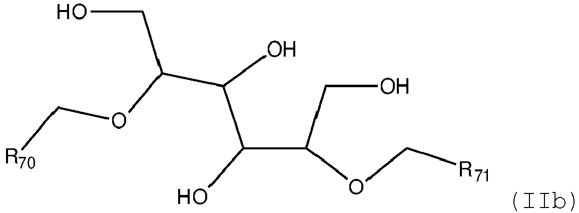 wherein R₇₀ an R₇₁ are selected from the group consisting of a linear or branched C₅ to C₂₀ alkyl, a (C₂ to C₁₀) -alkyloxy- (C₂ to C₁₀)-alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂)-alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIc) 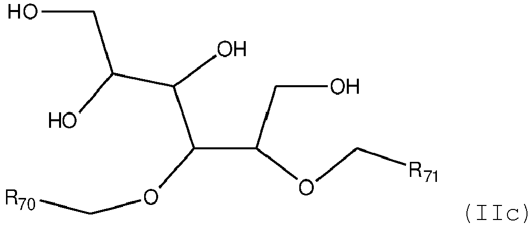 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IId) 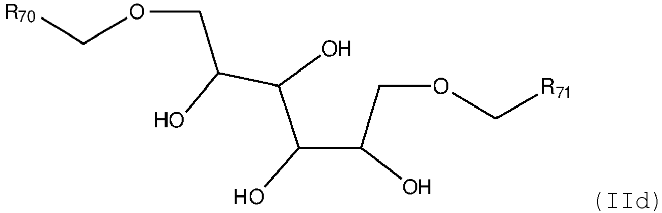 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIe) 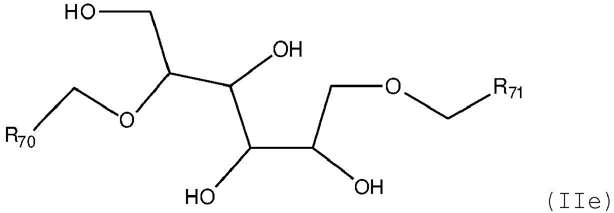 wherein R₇₀ an R₇₁ are selected from the group consisting of a linear or branched C₅ to C₂₀ alkyl, a (C₂ to C₁₀) -alkyloxy- (C₂ to C₁₀)-alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂)-alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIf) 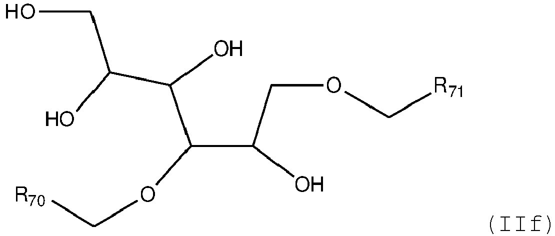 wherein R₇₀ an R₇₁ are selected from the group consisting of a linear or branched C₅ to C₂₀ alkyl, a (C₂ to C₁₀) -alkyloxy- (C₂ to C₁₀) -alkyl, a C₃ to C₁₀ cycloalkyl, cycloalkylalkyl, cycloalkylalkenyl and C₆ to C₁₂ aryl, and are preferably a linear C₅ to C₁₅ alkyl, C₂ to C₅)-alkyloxy-(C₁₀ to C₁₂) -alkyl, C₅-C₇ cycloalkyl and C₈ to C₁₀ aryl, and more preferably a linear C₈ to C₁₂ alkyl. Preferably, R₇₀ and R₇₁ are the same which allows to use the same starting compounds.

One embodiment of the present invention relates compound of the general formula (IIIa) 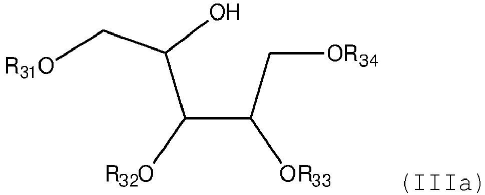 wherein R₃₁, R₃₂ and R₃₃ are hydrogen and R₇₁ is selected from the group consisting of a linear or branched C₅ to C₂₀ alkyl, preferably a linear C₈ to C₁₂ alkyl, and most preferably a linear C₁₂ alkyl.

The compounds of the present invention can be used as surfactants, preferably as detergents and additives in cleaning products. In particular the compounds of the general formula (Ia), (Ib), (Ic), (Id), (IIa), (IIb), (IIc), (IId), (IIe) and (IIf) are useful as emulsifiers, moisturizers, emollients, solubilizers, wetting agents, foam stabilizers in cosmetics products, food products, pharmaceutical products, in textile production processes for softening and helping the dye penetrate the fabric evenly and in the process of oil recovery and the remediation of petroleum oil contaminated soils. Said compounds are resistant towards the hydrolysis in alkaline media and provide antistatic properties. In addition, they are usually less aggressive than compounds carrying a sulphonate-group. Further they do not over-strip, i.e., they do not degrease to leave an excessively dry or squeaky' feel the skin or hair. Accordingly, said surfactants are especially preferred for surfactant-assisted synthesis of nanoparticles, oil-recovery with surfactant flooding, foam boosters and laundry applications. In addition, they are particularly useful as surfactants in oil recovery, in assisted synthesis of nanoparticle, as foam boosters and additives in cleaning formulations for laundry applications. In particular, it was found that said compounds provide low interfacial tensions and low microemulsion viscosities.

Furthermore, the compounds of the general formula (Ia), (Ib), (Ic), (Id), (IIa), (IIb), (IIc), (IId), (IIe) and (IIf) can be used as foam stabilizers in cosmetics products selected form the group consisting of soaps, shampooing formulations, creams, lotions, hair conditioners and cleansers. Preferably, said compounds are used in shampoos as foaming agent or detergent. Said shampoos contain significant proportions of said compounds in an aqueous medium which has preferably about a neutral pH. The shampoo has most preferably a pH in the range of 6.5 to 7.5, more preferably 6.8 to 7.3. Such shampoos have excellent foaming properties and a good rinsability. Furthermore, the foams provide a good stability.

The compounds of the general formula (Ia), (Ib), (Ic), (Id), (IIa), (IIb), (IIc), (IId), (IIe) and (IIf) can be used as food products selected from the group consisting of salad creams, dressings, sauces, desserts and anti-spattering agents for frying. The compounds of the present increase the shelf-life of the food products.

In a further embodiment of the present invention the compounds of the general formula (Ia), (Ib), (Ic), (Id), (IIa), (IIb), (IIc), (IId), (IIe) and (IIf) can be used as pharmaceutical products selected from the group consisting of drug-delivery systems, encapsulation of water-soluble vitamins in water-in-oil emulsion and encapsulation of oil-soluble vitamins in oil-in-water emulsion.

The compounds of the present invention can be easily obtained by the following reaction steps:

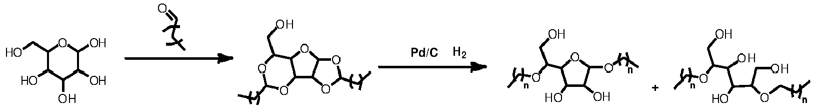

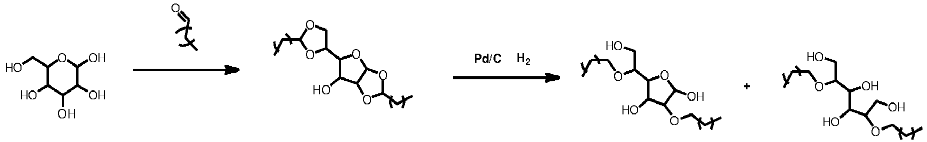

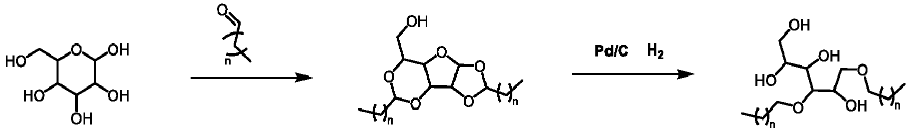

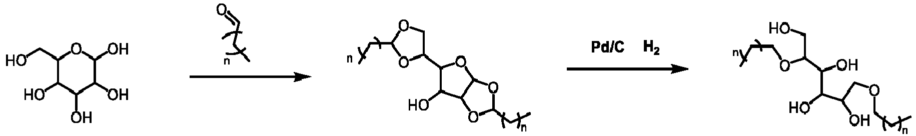

### Examples

### Synthesis of didodecylidene-xylose (8) from biomass

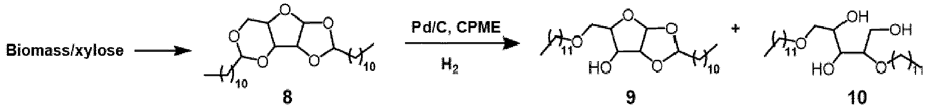

Biomass (5.00 g) was massed into a tared glass pressure bottle. To the reactor was then added the dodecanaldehyde (12.24g, 66 mmol), 1,4-dioxane (25 mL), hydrochloric acid (37 wt/wt%, 0.85 mL, 10 mmol), and a PTFE coated stir bar. The reaction was then heated with stirring to 85 °C for 3 hours. Once completed, the reactor was cooled down to room temperature and filtered through the Büchner funnel washing with dioxane (25 mL) to remove the cellulose-rich solids. The filtrate was then transferred to a round-bottom flask and concentrated on a rotavap. The concentrated crude reaction mixture was added dropwise using a glass pipette to an Erlenmeyer flask containing hexanes (250 mL) that was being stirred by a bar-type PTFE coated stir-bar at 250 RPM. Then filtration afforded precipitated lignin, and the filtrate can be concentrated on a rotavap and crystalized to obtain fully dodecanaldehyde-protected xylose.

### Synthesis of dodecyl-xylose ether (9,10)

Didodecylidene-xylose (8) was dissolved in cyclopentyl methyl ether (CPME) and together with 10% Pd/C catalyst were transferred to a 50-mL Parr reactor. The reactor was sealed and purged with hydrogen gas three times and hydrogen pressure was introduced (30 bar), and then heated to 135°C for 15 hours with stirring. The reactor was depressurized after cooled to room temperature and the reaction mixture was filtered. The filtrate was evaporated on a rotavap and the residue was purified by flash chromatography to give dodecyl-xylose ethers and acetals.

### Emulsion preparation

The emulsions were prepared by mixing 1ml of water (with 1mg/ml of Acian blue dye) and 2ml of cyclohexane containing 2-((dodecyloxy)methyl) tetrahydrofuran-3,4-diol at a concentration of 0.1% and then mixed by vortex for 30s.

### Emulsion stability measurements

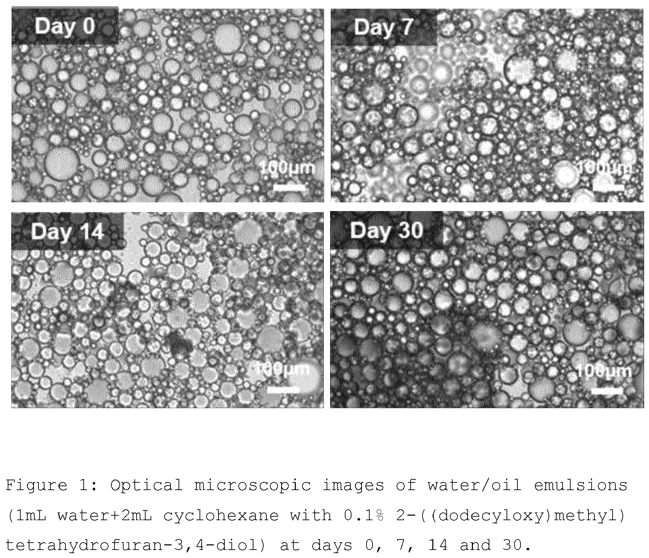

Emulsions were characterized using a bright-field microscope (Leitz Ergolux). Figure 1 shows that the aqueous bubbles in oil phase are stable for at least 30 days without obvious coalescence.

### Characterization data of xylose acetals and ethers 1,5-(decyloxy)pentane-2,3,4-triol (Ib)

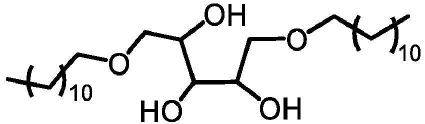

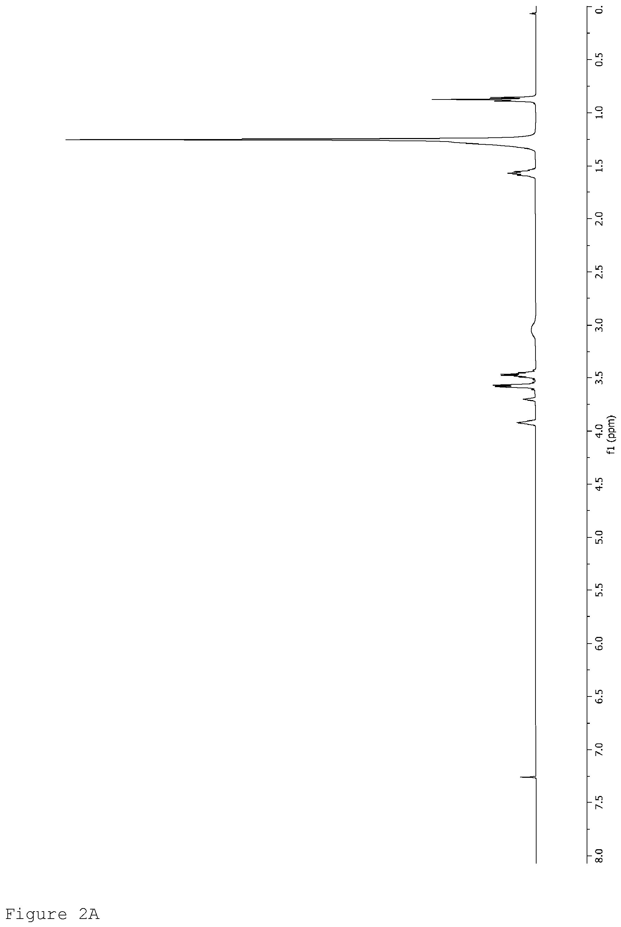

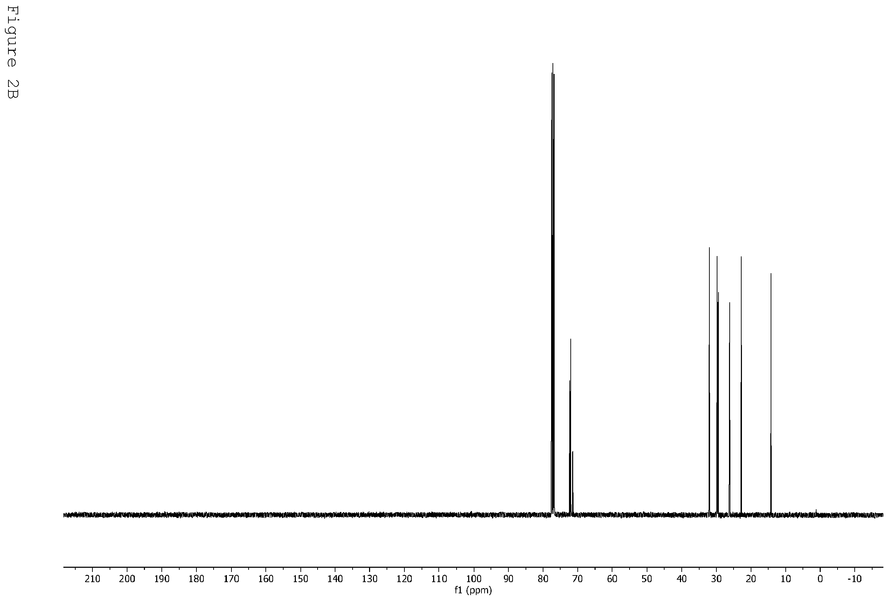

(c₁) ¹H NMR (400 MHz, CDC1₃) δ 3.86 (td, *J* = 5.2, 2.8 Hz, 2H), 3.64 (t, *J* = 2.9 Hz, 1H), 3.57 - 3.47 (m, 4H), 3.41 (td, *J* = 6.7, 3.7 Hz, 4H), 1.50 (q, *J* = 6.9 Hz, 4H), 1.13 - 1.30 (m, 36H), 0.81 (t, *J* = 6.8 Hz, 6H). Figure 2A ¹³C NMR (101 MHz, CDC1₃) δ 72.28, 72.02, 71.99, 71.41, 32.06, 29.81, 29.78, 29.75, 29.73, 29.71, 29.60, 29.49, 26.22, 22.83, 14.25. Figure 2B HRMS (ESI/QTOF) m/z: [M + Na]⁺ Calcd for C₂₉H₆₀NaO₅⁺ 511.4333; Found 511.4328.

### 3,5-(decyloxy)pentane-1,2,4-triol (Ic)

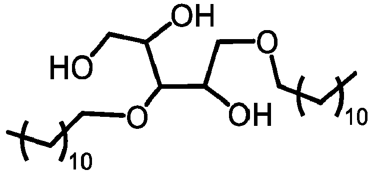

(c₂) ¹H NMR (400 MHz, CDCl₃) δ 4.15 (tt, *J* = 6.5, 5.5 Hz, 1H), 3.98 - 3.90 (m, 1H), 3.83 - 3.75 (m, 1H), 3.74 - 3.66 (m, 2H), 3.66 - 3.56 (m, 2H), 3.54 - 3.39 (m, 4H), 1.48-1.67 (m, 4H), 1.15-1.45 (m, 36H), 0.92 - 0.83 (m, 6H). ¹³C NMR (101 MHz, CDC1₃) δ 80.36, 72.86, 72.30, 72.03, 71.98, 71.78, 71.42, 70.56, 69.77, 60.87, 32.06, 30.17, 29.81, 29.78, 29.75, 29.73, 29.72, 29.62, 29.60, 29.49, 26.26, 26.24, 26.22, 22.83, 14.26.

HRMS (ESI/QTOF) m/z: [M + Na]⁺ Calcd for C₂₉H₆₀NaO₅⁺ 511.4333; Found 511.4335.

### 5-(dodecyloxy)pentane-1,2,3,4-tetraol (IIIa)

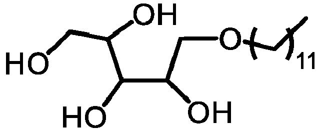

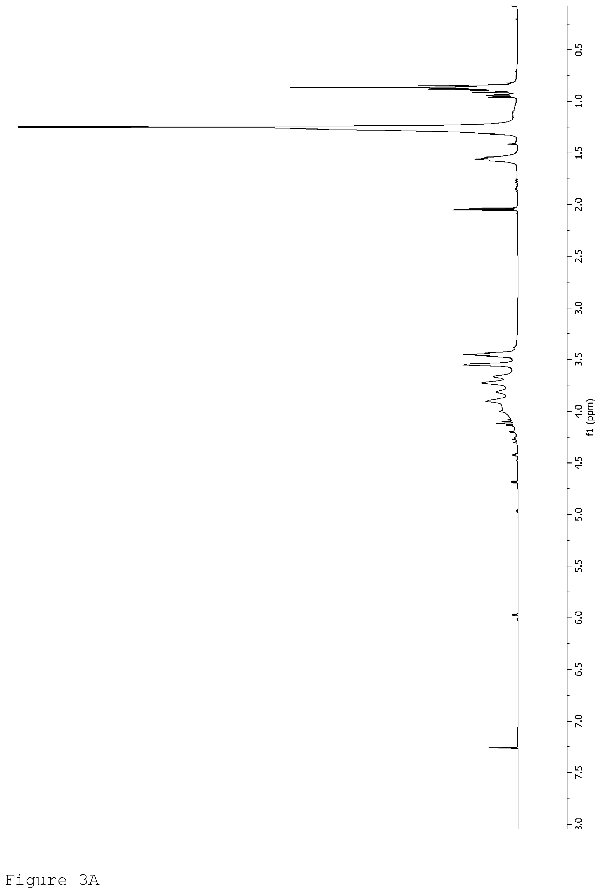

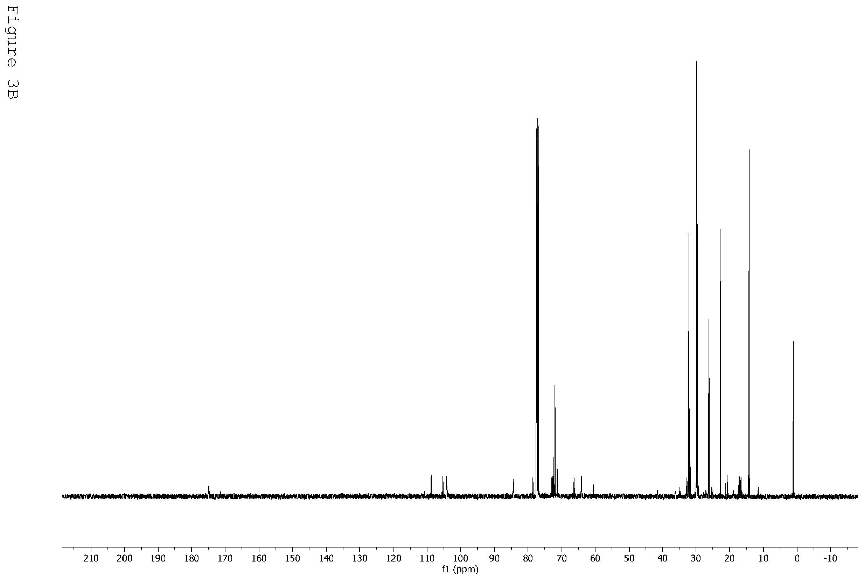

¹H NMR (400 MHz, CDC1₃) 3.86 - 3.73 (m, 2H), 3.70 (dd, *J* = 11.6, 5.2 Hz, 1H), 3.63 - 3.40 (m, 4H), 3.20 (td, *J* = 7.3, 5.2 Hz, 1H), 2.87 (t, *J* = 5.5 Hz, 1H), 1.60 - 1.50 (m, 2H), 1.34 - 1.22 (m, 18H), 0.92 - 0.83 (m, 3H). Figure 3A ¹³C NMR (101 MHz, CDC1₃) δ 75.23, 72.77, 72.52, 72.27, 72.10, 71.82, 71.09, 64.58, 35.71, 33.97, 32.05, 31.77, 29.80, 29.79, 29.74, 29.72, 29.70, 29.68, 29.65, 29.49, 29.18, 29.05, 26.22, 22.73, 14.19. Figure 3B HRMS (LTQ-Orbitrap) m/z: [M + H]⁺ Calcd for C₁₇H₃₇O₅⁺ 321.2636; Found 321.2632.

### Interfacial tension measurements

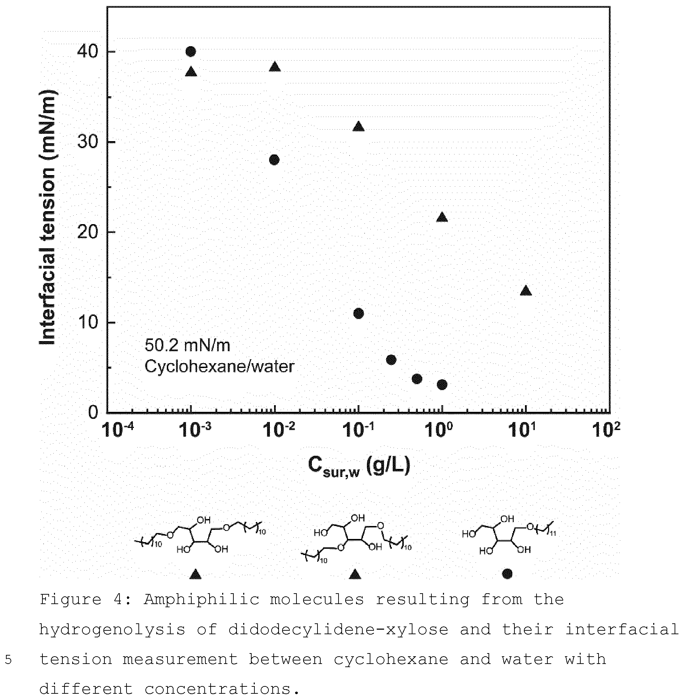

To test the amphiphilic properties of each molecule, we measured the water/oil interfacial tension using the pendant drop test. In a typical test, we slowly inject the organic solution containing the surfactants into the water phase with a bent needle of 1mm of diameter. The interfacial tension is then calculated by the force balance with the buoyancy force. The critical micelle concentration (CMC) is obtained by checking the critical point of interfacial tension-concentration graph. Figure 4 shows that the CMC of 5-(dodecyloxy)pentane-1,2,3,4-tetraol (f) is around 1g/L and induces a decrease of the interfacial tension (cyclohexane/water) to a plateau value about 2.7 mN/m.

## Claims

1. Compound of the general formula (I), (II), and (III) 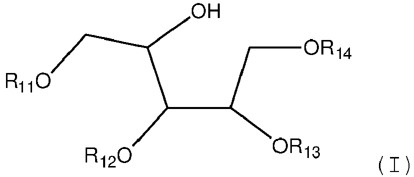 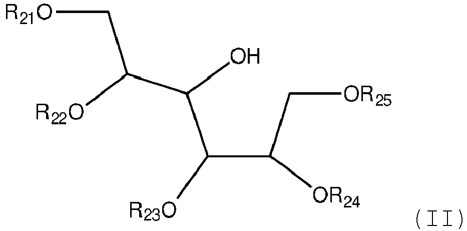 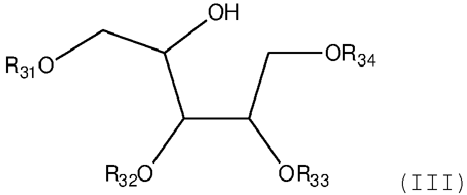
wherein one of R₁₁ and R₁₂ is hydrogen and the other is -CH₂-R₇₀, and one of R₁₃ and R₁₄ is hydrogen and the other is -CH₂-R₇₁, or wherein one of R₁₁ and R₁₂ is hydrogen and the other is -CH₂-R₇₀, and both of R₁₃ and R₁₄ are hydrogen, and
wherein two of R₂₁, R₂₂ and R₂₃ is hydrogen and the other is -CH₂-R₇₀, and one of R₂₄ and R₂₅ is hydrogen and the other is -CH₂-R₇₁, and
wherein R₃₁, R₃₂ and R₃₃ are hydrogen and R₃₄ is -CH₂-R₇₁, and
R₇₀ and R₇₁ are independently form each other and are selected from the group consisting of a linear or branched C₅ to C₂₀ alkyl, a (C₂ to C₁₀) -alkyloxy- (C₂ to C₁₀)-alkyl, a C₅ to C₁₀ cycloalkyl and C₆ to C₁₂ aryl.

2. Compound of the general formula (I), and (II) according to Claim 1, wherein R₇₀ and R₇₁ are the same.

3. Compound of the general formula (Ia) according to Claim 1 or 2, 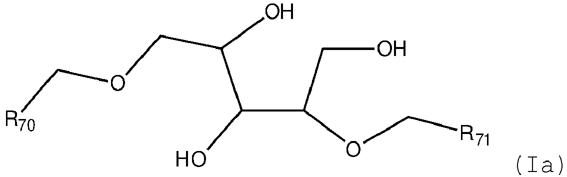 wherein R₇₀ an R₇₁ are selected from the group consisting of a linear or branched C₅ to C₂₀ alkyl, a (C₂ to C₁₀)-alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

4. Compound of the general formula (Ib) according to Claim 1 or 2, 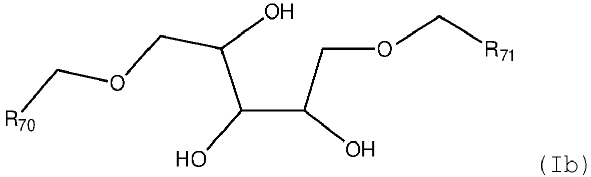 wherein R₇₀ and R₇₁ are selected from the group consisting of a linear or branched C₅ to C₂₀ alkyl, a (C₂ to C₁₀) - alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

5. Compound of the general formula (Ic) according to Claim 1 or 2, 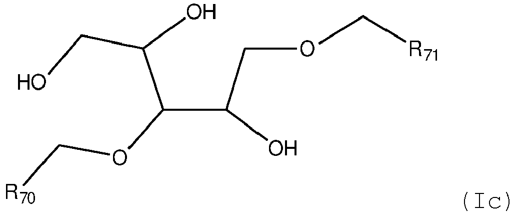 wherein R₇₀ and R₇₁ are selected from the group consisting of a linear or branched C₅ to C₂₀ alkyl, a (C₂ to C₁₀)-alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

6. Compound of the general formula (Id) according to Claim 1 or 2, 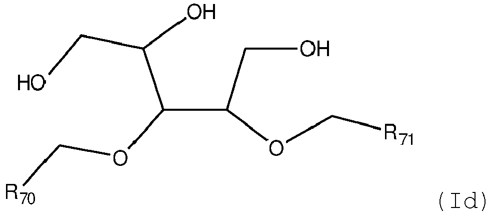 wherein R₇₀ and R₇₁ are selected from the group consisting of a linear or branched C₅ to C₂₀ alkyl, a (C₂ to C₁₀) - alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

7. Compound of the general formula (IIa) according to Claim 1 or 2, 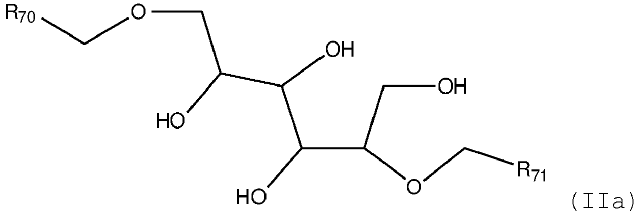 wherein R₇₀ and R₇₁ are selected from the group consisting of a linear or branched C₅ to C₂₀ alkyl, a (C₂ to C₁₀)-alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

8. Compound of the general formula (IIb) according to Claim 1 or 2, 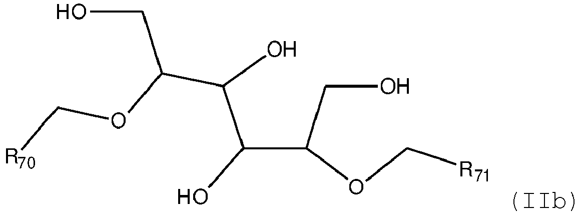 wherein R₇₀ and R₇₁ are selected from the group consisting of a linear or branched C₅ to C₂₀ alkyl, a (C₂ to C₁₀) - alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

9. Compound of the general formula (IIc) according to Claim 1 or 2, 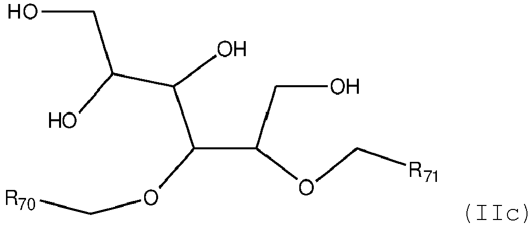 wherein R₇₀ and R₇₁ are selected from the group consisting of a linear or branched C₅ to C₂₀ alkyl, a (C₂ to C₁₀) - alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

10. Compound of the general formula (IId) according to Claim 1 or 2, 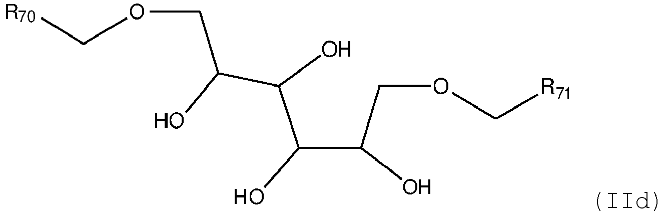 wherein R₇₀ and R₇₁ are selected from the group consisting of a linear or branched C₅ to C₂₀ alkyl, a (C₂ to C₁₀) - alkyloxy- (C₂ to C₁₀)-alkyl, a C₅ to C₁₀ cycloalkyl and C₆ to C₁₂ aryl and are preferably a linear C₅ to C₁₅ alkyl and more preferably a linear C₈ to C₁₂ alkyl.

11. Compound of the general formula (IIe) according to Claim 1 or 2, 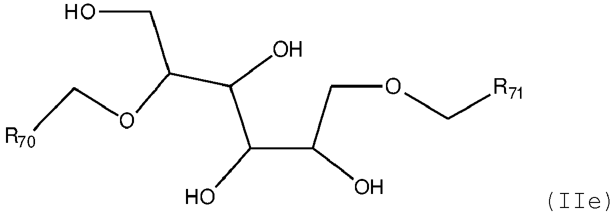 wherein R₇₀ and R₇₁ are selected from the group consisting of a linear or branched C₅ to C₂₀ alkyl, a (C₂ to C₁₀) - alkyloxy- (C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

12. Compound of the general formula (IIf) according to Claim 1 or 2, 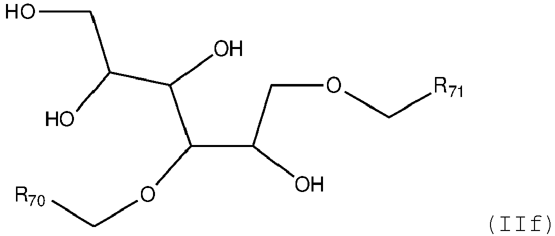 wherein R₇₀ and R₇₁ are selected from the group consisting of a linear or branched C₅ to C₂₀ alkyl, a (C₂ to C₁₀) - alkyloxy-(C₂ to C₁₀) -alkyl, a C₅ to C₁₀ cycloalkyl and C₆ to C₁₂ aryl and are preferably a linear C₅ to C₁₅ alkyl and more preferably a linear C₈ to C₁₂ alkyl.

13. Use of the compounds of the general formula (I), (II) and (III) according to any of Claims 1 to 12 as surfactants.

14. Use of the compounds of the general formula (Ia), (Ib), (Ic), (Id), (IIa), (IIb), (IIc), (IId), (IIe) and (IIf) according to any of Claims 3 to 12 as emulsifiers, moisturizers, emollients, solubilizers, wetting agents, foam stabilizers in cosmetics products, food products, pharmaceutical products, in textile production processes, in the process of oil recovery and the remediation of petroleum oil contaminated soils.

15. Use of the compounds of the general formula (Ia), (Ib), (Ic), (Id), (IIa), (IIb), (IIc), (IId), (IIe) and (IIf) according to any of Claims 3 to 12 as foam stabilizers in cosmetics products selected form the group consisting of soaps, shampooing formulations, creams, lotions, hair conditioners and cleansers or as food products selected from the group consisting of salad creams, dressings, sauces, desserts and antispattering agents for frying or as pharmaceutical products selected from the group consisting of drug-delivery systems, encapsulation of water-soluble vitamins in water-in-oil emulsion and encapsulation of oil-soluble vitamins in oil-in-water emulsion.
